**Europäisches Patentamt**

**European Patent Office**

**Office européen des brevets**

(19)

(11) Veröffentlichungsnummer : **0 438 453 B1**

(12)
# EUROPÄISCHE PATENTSCHRIFT

(45) Veröffentlichungstag der Patentschrift :
**18.08.93 Patentblatt 93/33**

(51) Int. Cl.$^5$ : **A61M 5/50**

(21) Anmeldenummer : **89911352.6**

(22) Anmeldetag : **13.10.89**

(86) Internationale Anmeldenummer :
**PCT/AT89/00090**

(87) Internationale Veröffentlichungsnummer :
**WO 90/03817 19.04.90 Gazette 90/09**

(54) INJEKTIONSVORRICHTUNG MIT ORIGINALITÄTSSICHERUNGSVORRICHTUNG.

(30) Priorität : **13.10.88 AT 2549/88**
**11.05.89 AT 1131/89**

(43) Veröffentlichungstag der Anmeldung :
**31.07.91 Patentblatt 91/31**

(45) Bekanntmachung des Hinweises auf die
Patenterteilung :
**18.08.93 Patentblatt 93/33**

(84) Benannte Vertragsstaaten :
**AT BE CH DE FR GB IT LI LU NL SE**

(56) Entgegenhaltungen :
**EP-A-02 911 09**

(56) Entgegenhaltungen :
**FR-A-22 983 40**
**FR-A-26 066 43**
**GB-A-22 070 54**
**US-A- 3 325 062**

(73) Patentinhaber : **PICKHARD, Ewald**
**Redtenbachergasse 15**
**A-1160 Wien (AT)**

(72) Erfinder : **PICKHARD, Ewald**
**Redtenbachergasse 15**
**A-1160 Wien (AT)**

(74) Vertreter : **Secklehner, Günter, Dr.**
**Rechtsanwalt**
**Pyhrnstrasse 1**
**A-8940 Liezen (AT)**

EP 0 438 453 B1

Jouve, 18, rue Saint-Denis, 75001 PARIS

## Beschreibung

Die Erfindung betrifft eine Injektionsvorrichtung mit einem Spritzenzylinder, einem in dem Spritzenzylinder geführten Kolben und einer mit diesem insbesondere über eine Kupplungsvorrichtung verbundene Kolbenstange, die bevorzugt eine Länge aufweist, die größer ist als die Länge des Spritzenzylinders, mit einer auf der von der Kolbenstange abgewendeten Seite des Spritzenzylinders vorgesehenen Kupplungsanordnung, über die eine Injektionsnadel mit dem Spritzenzylinder kuppelbar ist und mit einer originalitätssicherungsvorrichtung, die ein zwischen dem Kolben und der Kolbenstange angeordnetes, durch einen Öffnungsdorn gebildetes Öffnungsorgan und der Kolben eine parallel zur Längsachse des Spritzenzylinders verlaufende Bohrung aufweist. Eine bekannte Injektionsvorrichtung für den Einmalgebrauch - gemäß EP-A-0 291 109 - weist zwischen einem Kolben und einer Kolbenstange eine Kupplunganordnung mit einer Auslöseeinrichtung auf, die eine Relativbewegung zwischen Kolben und Kolbenstange bei einer Wiederverwendung bewirkt. Durch diese Relativbewegung wird ein Öffnungskanal im Kolben freigelegt, der die Druckkammer des Spritzenzylinders mit der Umgebungsluft verbindet, der die Bildung eines Vakuums zum Aufsaugen eines Medikamentes verhindert. Bei einem vorsichtigen Gebrauch dieser Injektionsvorrichtung ist jedoch ein Mehrmalgebrauch bei einer über die Injektionsnadel erfolgenden Druckbefüllung der Druckkammer des Spritzenzylinders nicht gänzlich auszuschließen.

Eine andere bekannte Injektionsvorrichtung - gemäß GB-A-2 207 054 - die nur zum Einmalgebrauch geeignet ist, weist einen Spritzenzylinder, einen in den Spritzenzylinder geführten Kolben und eine über eine Kupplungsvorrichtung mit dem Kolben verbundene Kolbenstange auf. Auf der von der Kolbenstange abgewendeten Seite des Spritzenzylinders ist die Injektionsnadel über eine Kupplungsanordnung mit dem Spritzenzylinder gekuppelt. Um die Einmalverwendung dieser Injektionsvorrichtung sicher zu stellen ist diese mit einer originalitätssicherungsvorrichtung ausgerüstet, die ein zwischen dem Kolben und der Kolbenstange angeordnetes Öffnungsorgan aufweist. Dieses Öffnungsorgan wird durch einen mit der Kolbenstange verbundenen Stoppel gebildet, der bei dem Auspressen der Injektionsflüssigkeit durch den Kolben hindurchgedrückt werden soll, um eine Verbindung zwischen dem die Injektionsflüssigkeit aufnehmenden Innenraum und der Umgebungsluft herzustellen, sodaß ein weiteres Ansaugen eines Medikamentes nicht mehr möglich ist. Des weiteren wird auch vorgeschlagen, den Kolben an seiner Stirnseite mit einer verformbaren Membran auszustatten, die bei Druckbeaufschlagung des Kolbens zum Auspressen der Injektionsflüssigkeit gegen einen Öffnungsdorn gepreßt wird. Dadurch soll die dichtende Membran zerstört und das Aufziehen eines weiteren Medikamentes mit der Injektionsvorrichtung verhindert werden. Nachteilig ist hierbei, daß bereits bei einer geringfügigen Fehlbedienung die Injektionsvorrichtung beispielsweise während des Aufziehens der Injektionsflüssigkeit zerstört werden kann und dadurch unbrauchbar wird.

Es sind bereits verschiedene Injektionsspritzen bekannt, die mit unterschiedlichen Sicherheitsvorrichtungen versehen werden können, um eine einmalige Verwendung der Injektionsvorrichtung sicherzustellen. So ist es, um eine Mehrfachverwendung zu vermeiden, bereits bekannt - gemäß GB-OS 2 195 537 des gleichen Anmelders - die Injektionsnadel mit einem Originalitätsverschluß, beispielsweise einer Schutzhülle, zu umgeben. Bei einer Verwendung einer Injektionsspritze muß nun dieser Originalitätsverschluß und die Schutzkappe entfernt werden, wodurch nach dem Gebrauch der Injektionsvorrichtung die Zerstörung des Originalitätsverschlusses jederzeit erkennbar und somit feststellbar ist, ob die Injektionsvorrichtung bereits verwendet wurde oder nicht. Zudem ist es auch bereits bekannt, in den Spritzen selbst, beispielsweise zwischen den Spritzennadeln und den Spritzenzylindern durch Membranen oder dgl., gebildete Originalitätsverschlüsse vorzusehen, die erst unmittelbar vor der Verwendung der Injektionsvorrichtung durchstoßen werden, sodaß das Medikament bis zur tatsächlichen Verwendung luftdicht verschlossen in dem Spritzenzylinder untergebracht ist. Nachteilig ist auch bei diesen Injektionsvorrichtungen, daß nach dem Ausspritzen des Medikamentes aus dem Spritzenzylinder durch Betätigung des Spritzenkolbens ein weiteres Medikament in den Spritzenzylinder eingesaugt werden kann und damit die Injektionsvorrichtung in unerlaubter Weise ein zweites Mal verwendet werden kann. Dies stellt insbesondere in Ländern mit geringem Hygienebewußtsein eine große Gefahr dar, die vielfach zu einer epidemischen Verbreitung von meist ansteckenden Krankheiten, insbesondere Aids führt.

Eine bekannte Injektionsvorrichtung - gemäß FR-A-2 606 643 - beschreibt eine Injektionsvorrichtung mit einem Spritzenzylinder, in dem ein Kolben geführt ist, der mit einer Kolbenstange verbunden ist. Die Kolbenstange weist eine Länge auf, die größer ist als die Länge des Spritzenzylinders. Auf der von der Kolbenstange abgewendeten Seite des Spritzenzylinders ist eine Kupplungsanordnung vorgesehen, über die eine Injektionsnadel mit dem Spritzenzylinder kuppelbar ist. Im Bereich einer der Injektionsnadel zugewandten Stirnwand des Spritzenzylinders ist eine originalitätssicherungsvorrichtung vorgesehen, die ein mit dem Spritzenzylinder verbundenes, in Richtung des Kolben weisendes Öffnungsorgan aufweist. Diesem Öfnungorgan ist im Bereich des Kolbens ein Verschlußelement bzw. eine Membran zugeordnet. Wird nun der Kolben mit der Kolbenstange bis zum Anschlag an die Stirnwand des Spritzenzylinders vorwärtsbewegt, so wird durch das Öffnungsorgan

2

das Verschlußelement geöffnet bzw. die Membran zerstört. Durch die solcherart erfolgte Zerstörung des Kolbens ist es nicht mehr möglich, ein weiteres Injektionsmedium in dem Spritzenzylinder aufzuziehen. Damit ist die Injektionsvorrichtung für eine Mehrfachverwendung zerstört. Nachteilig ist hierbei, daß bei nicht völliger Entleerung des Spritzenzylinders, also dann, wenn der Kolben nicht bis zum Anschlag an die Stirnwand des Spritzenzylinders vorbewegt wird, sehr wohl mehrfach hintereinander Injektionsflüssigkeiten aufgezogen und mehreren Patienten verabreicht werden kann. Damit kann eine Mehrfachverwendung der Injektionsvorrichtung nicht zuverlässig verhindert werden.

Der vorliegenden Erfindung liegt nunmehr die Aufgabe zugrunde, eine Injektionsvorrichtung zu schaffen, die auch nach dem Ausspritzen einer geringen Menge eines Medikamentes nicht mehr zum Einfüllen eines weiteren oder neuen Medikamentes verwendet werden kann.

Diese Aufgabe der Erfindung wird durch die Merkmale im Patentanspruch 1 gelöst. Der Vorteil dieser Originalitätssicherungsvorrichtung liegt darin, daß bewegungsbedingt ohne irgendwelche Hilfsmittel, beispielsweise Speicherelemente wie vorgespannten Ringen oder dgl., die Injektionsvorrichtung unbrauchbar gemacht bzw. zerstört wird, sodaß eine Wiederverwendung zuverlässig unterbunden ist. Durch die Verwendung eines Öffnungsorgans, das mit dem Kolben bzw. der Kolbenstange verbunden oder zwischen diesen angeordnet ist, kann bei einer beliebig vorwählbaren Stellung zwischen Kolbenstange und Kolben unabhängig von der Höhe des Kolbenhubes im Spritzenzylinder die Injektionsvorrichtung unbrauchbar gemacht werden. Durch die Betätigung des Innenkolbens zum Vorwärtsschieben des Kolbens wird nämlich bereits beim erstmaligen Auspressen der Lösung aus dem Innenraum der Injektionsvorrichtung mit dem Öffnungsdorn das Verschlußelement durchstochen, sodaß auch eine Mehrfachverwendung dann nicht möglich ist, wenn nicht der gesamte Lösungsinhalt aus dem Innenraum des Spritzenzylinders ausgepreßt wird.

Durch die Verwendung des weiteren Kolbens, also einer sogenannten Doppelkolbenanordnung kann in vorteilhafter Weise bei entsprechender Ausbildung und Anordung der Bypaßleitung mit dem im Kolben geführten Innenkolben eine geringe Lösungsmenge auch nach dem Zerstören des Verschlußelementes noch angesaugt werden, wie dies beispielsweise beim sogenannten Aspirieren notwendig ist. Trotzdem ist aber erreicht, daß das Aufziehen einer größeren Lösungsmenge in den Innenraum des Spritzenzylinders nur durch eine Betätigung des den Innenkolben aufnehmenden Kolben möglich ist.

Da jedoch durch die Verwendung eines mit dem Verschlußelement zusammenwirkenden Öffnungsdorns beim Zurückziehen des Innenkolbens beispielsweise zum Aufziehen einer weiteren Lösung zwangsweise eine Verbindung des Innenraums mit der Umgebungsluft hergestellt wird, kann im Innenraum kein Unterdruck zum Aufziehen einer weiteren Lösung hergestellt werden und ist dementsprechend die Injektionsvorrichtung unbrauchbar gemacht ist.

Weiters ist auch eine Ausbildung nach Patentanspruch 2 möglich. Dadurch ist in einfacher Weise sichergestellt, daß bei zerstörtem Verschlußelement und einer Stellung des Innenkolbens zum Aufziehen einer weiteren Lösung kein Unterdruck im Innenraum der Injektionsvorrichtung mehr aufgebaut werden kann.

Nach Patentanspruch 3 ist eine andere Ausführungsform vorgesehen, wodurch ein sicherer und dichter Abschluß des Innenraumes beim Aufziehen der ersten zu injizierenden Lösung sichergestellt und andererseits eine einfache Zerstörung beim Auspressen dieser ersten Lösung aus dem Innenraum erreicht werden kann.

Es ist aber auch eine Ausgestaltung nach Patentanspruch 4 möglich, wodurch ein dichter Abschluß des Innenraums auch über eine lange Lagerdauer möglich ist und andererseits nach dem Durchstoßen des Verschlußelementes mit dem Öffnungsdorn eine verbleibende Öffnung sichergestellt ist.

Es ist aber auch eine Lösung nach Patentanspruch 5 möglich, wodurch die Dichtscheibe erst bei einem größeren Kraftaufwand, wie er beispielsweise dazu notwendig ist, um eine Lösung in einen menschlichen oder tierischen Körper zu injizieren, ausgestoßen wird.

Eine Verzwängungen des Kolbens und damit eine erschwerte Bedienung der Injektionsvorrichtung wird durch die Ausführung nach Patentanspruch 6 verhindert.

Vorteilhaft ist eine Weiterbildung nach Patentanspruch 7. Dadurch wird eine einwandfreie Funktion des Öffnungsdorns auch beim Aufbringen von hohen Druckkräften zum Durchstechen des Verschlußelementes sichergestellt.

Vorteilhaft ist eine Weiterbildung nach Patentanspruch 8. Dadurch können unerwünschte Luftblasen zwischen dem Verschlußelement und dem Innenkolben zuverläßig verhindert werden. Wird das Spreizorgane des Verschlußelementes vom Öffnungsdorn abgehoben, kann die Lösung zuverläßig zwischen das Verschlußelement und die Innenfläche des Kolbens eintreten und die dort vorhandene Luft auspressen.

Eine andere Ausbildung ist nach Patentanspruch 9 vorgesehen, wodurch das Kippen des Kolbens und damit eine Zerstörung der Injektionsvorrichtung während des erstmaligen Gebrauches verhindert wird.

Eine höhere Dichtheit und somit ein höherer Auspreßdruck kann durch die Ausführung nach Patentanspruch 10 erreicht werden.

Weiters ist auch die Ausgestaltung nach Patentanspruch 11 möglich, wodurch beim Auspressen der Lösung bei der erstmaligen Verwendung ein unerwünschter Durchtritt desselben in Richtung der Kolbenstange und damit ein Lösungsverlust verhindert werden kann.

Vorteilhaft ist aber auch die Lösung nach Patentanspruch 12. Damit ist sichergestellt, daß beim Durchstoßen des Verschlußelementes bzw. bei dessen Entfernung dieses nicht in den Innenraum eintreten und damit den Austritt der Lösung durch die Injektionsnadel verhindern kann.

Eine andere Ausführungsvariante ist nach Patentanspruch 13 ausgebildet. Diese Ausführungsform bietet den zusätzlichen Vorteil, daß je nach der Distanz zwischen den Vorsprüngen des Kolbens und der Vertiefung im Spreizring ein ungehindertes Vor- und Zurückbewegen des Kolbens möglich ist, wodurch beispielsweise ein Aspirieren mit einer derart ausgebildeten Injektionsvorrichtung möglich ist, jedoch beim vollständigen Auspressen der Lösung aus dem Innenraum der Injektionsvorrichtung die Rastarme mit ihren Arretiernasen in die Vertiefungen des Spreizringes einrasten und somit in einer Öffnungsstellung gehalten werden, die ein Wiedereinkuppeln der Arretiernasen der Rastarme in die Vorsprünge auf der Rückseite des Kolbens zuverlässig verhindern soll und damit ebenfalls eine Mehrfachverwendung der Injektionsvorrichtung ausgeschlossen ist.

Eine andere Weiterbildung beschreibt Patentanspruch 14. Es wird eine versehentliche Zerstörung der Injektionsvorrichtung vor der erstmaligen Benutzung verhindert und mit Sicherheit in jedem Fall ein Lösen der Kolbenstange vom Kolben nach dem Auspressen einer bestimmten Lösungsmenge erreicht. Ein darüber hinausgehender Vorteil liegt darin, daß für den Fall eines versehentlichen Zurückziehens der Kolbenstange und einer damit einhergehenden Entkupplung zwischen dieser und dem Kolben, wie dies beispielsweise bei einem zu weiten Zurückziehen beim Aspirieren der Fall sein könnte, die Kolbenstange wieder eingeführt und zumindest eine Druckkraft zum Auspressen der Lösung aus der Injektionsvorrichtung aufgebracht werden kann. Bei einem erneuten Versuch, den Kolben mit der Kolbenstange zurückzuziehen entkuppelt sich die Kolbenstange wieder selbsttätig vom Kolben und die Injektionsvorrichtung ist für eine Weiterverwendung unbrauchbar.

Eine vorteilhafte Weiterbildung ist im Patentanspruch 15 enthalten. Dadurch wird auch bei Fertigungstoleranzen ein sicheres Entkuppeln der Arretiernasen der Rastarme sichergestellt.

Eine andere Ausführungsvariante ist nach Patentanspruch 16 möglich, wodurch das Einführen der Steuerleisten in die Durchgangsöffnung vor dem Durchtreten der Steuerleisten durch diese begünstigt und somit eine sichere Auslösefunktion erzielt wird.

Eine andere Weiterbildung beschreibt Patentanspruch 17. Dadurch wird auch bei den durch die industrielle Herstellung nicht ganz zu vermeidenden Toleranzen ein sicheres Durchstoßen des Verschlußelementes erreicht und eine sichere Funktion der Injektionsvorrichtung gewährleistet.

Zum besseren Verständnis der Erfindung wird diese im nachfolgend anhand der in den Zeichnungen gezeigten Ausführungsbeispiele näher erläutert.

Es zeigen:

Fig. 1 eine erfindungsgemäß ausgebildete Injektionsvorrichtung in Seitenansicht, geschnitten und stark vereinfachter schematischer Darstellung;

Fig. 2 die Injektionsvorrichtung nach Fig.1, nachdem die in die Injektionsvorrichtung aufgenommene Lösung teilweise ausgepreßt wurde mit einer anderen Ausführungsform eines Innenkolbens;

Fig. 3 eine andere Ausführungsform einer erfindungsgemäßen Injektionsvorrichtung in Seitenansicht, geschnitten, gemäß den Linien III-III in Fig.4;

Fig. 4 die Injektionsvorrichtung in Stirnansicht, geschnitten, gemäß den Linien IV-IV in Fig.3;

Fig. 5 die Injektionsvorrichtung nach Fig.3 während des Aufziehens einer Lösung in den Innenraum der Injektionsvorrichtung in Seitenansicht, geschnitten;

Fig. 6 die Kolbenstange der Injektionsvorrichtung nach den Fig.3 bis 5 in Stirnansicht, geschnitten, gemäß den Linien VI-VI in Fig.5;

Fig. 7 die Injektionsvorrichtung nach den Fig.3 bis 6 in einer Stellung beim Auspressen der Lösung aus dem Innenraum der Injektionsvorrichtung und bei zerstörtem Öffnungselement;

Fig. 8 eine andere Ausführungsvariante einer erfindungsgemäß ausgebildeten Injektionsvorrichtung in Seitenansicht, geschnitten in ihrer Stellung beim Aufziehen einer Lösung in den Innenraum;

Fig. 9 die Injektionsvorrichtung nach Fig.8 in ihrer Stellung beim Aspirieren bzw. Auspressen der Lösung aus dem Innenraum;

Fig. 10 die Injektionsvorrichtung nach den Fig.8 und 9 während des Aspiriervorganges beim Zurückziehen der Kolbenstange;

Fig. 11 eine andere Ausführungsvariante einer erfindungsgemäßen Injektionsvorrichtung mit einem zweiteiligen Innenkolben in seiner Stellung beim Aufziehen der Lösung in den Innenraum der Injektionsvorrichtung in Seitenansicht, geschnitten;

Fig. 12 die Injektionsvorrichtung nach Fig.11 in ihrer Stellung beim Auspressen der Lösung aus dem Innenraum und beim erneuten Versuch des Aufziehens einer weiteren Lösung in Seitenansicht, geschnitten und stark vereinfachter schematischer Darstellung;

Fig. 13   den vorderen Abschnitt einer weiteren Ausführungsform einer erfindungsgemäßen Injektionsvorrichtung im Schnitt ;

Fig. 14   eine Darstellung analog zu Fig.13 mit einer abgewandelten Ausführungsform einer erfindungsgemäßen Injektionsvorrichtung;

Fig. 15   einen Teilschnitt durch den vorderen Abschnitt einer weiteren Ausführungsform einer erfindungsgemäßen Injektionsvorrichtung mit einem Dichtungskolben;

Fig. 16   einen Teilschnitt durch den vorderen Abschnitt einer weiteren Ausführungsform einer erfindungsgemäßen Injektionsvorrichtung mit einem Dichtungskolben;

Fig. 17   einen Schnitt analog zu den Fig.13 und 14 durch eine abgewandelte Ausführungsform der erfindungsgemäßen Injektionsvorrichtung mit einer im Spritzenkolben verschieblich geführten Schneide;

Fig. 18   eine Ansicht in Richtung des Pfeiles XVIII in Fig.17 auf die den Spritzenkonus tragende Stirnwand der Einwegspritze;

Fig. 19   eine Ausführungsform einer erfindungsgemäßen Injektionsvorrichtung mit einem am kolbenseitigen Ende des Spritzenkonus über radiale Stege abgestützten Dorn;

Fig. 20   eine Ansicht auf die Abstützung des Dornes gemäß Fig.19.

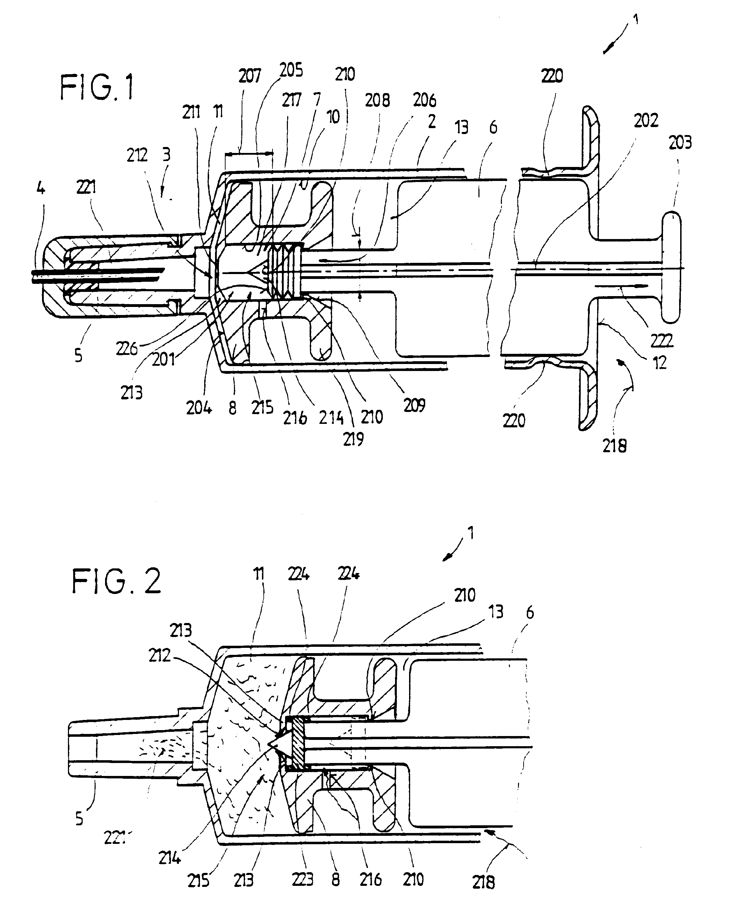

In Fig.1 ist eine Injektionsvorrichtung 1 gezeigt, die aus einem Spritzenzylinder 2 und einer über eine Kupplungsanordnung 3 mit dieser verbundenen Injektionsnadel 4 besteht. Die Kupplungsanordnung 3 ist auf einem rohrförmigen Auslaß 5 der Spritzenzylinder 2 angeordnet. Im Inneren des Spritzenzylinders 2 ist in dessen Längsrichtung verschiebbar eine Kolbenstange 6 angeordnet, die über eine Kupplungsvorrichtung 7 mit einem Kolben 8 bewegungsverbunden ist. Der Kupplungsvorrichtung 7 ist eine Originalitätssicherungsvorrichtung 201 zugeordnet. Der Kolben 8 kann aus einem Gummi- oder Kunststoffteil oder einem entsprechenden Kunststoffeinsatz bestehen, an dessen Umfang Dichtelemente, wie beispielsweise 0-Ringe oder Gummimanschetten oder dgl., angeordnet sind. Mit diesen Gummimanschetten bzw. 0-Ringen liegt der Kolben 8 an einer Innenwand 10 des Spritzenzylinders 2 an und unterteilt diese in eine mit dem Auslaß 5 in Verbindung stehende Zylinderkammer 11 und eine einer Öffnung 12 des Spritzenzylinders 2 zugeordnete Zylinderkammer 13.

Zum Betätigen der Kolbenstange 6 und zum Verschieben derselben entlang einer Längsachse 202 des Spritzenzylinders 2 ist sie mit einer Handhabe 203 versehen, die, wie aus dem dargestellten Ausführungsbeispiel ersichtlich, bei einer Stellung, in der eine Stirnfläche 204 des Kolbens 8 an der der Injektionsnadel 4 zugewandten Stirnwand des Spritzenzylinders 2 anliegt, die Handhabe 203 noch aus dem Spritzenzylinder 2 herausragt. Die dargestellte Stellung entspricht auch dem Verpackungszustand einer erfindungsgemäß ausgebildeten Injektionsvorrichtung 1, wobei in diesem Fall die Injektionsnadel 4 noch nicht auf den Auslaß 5 aufgeschoben ist.

Im Kolben 8 ist eine Bohrung 205 angeordnet, in der ein Innenkolben 206 in Richtung der Längsachse 202 verschiebbar gelagert ist. Der Innenkolben 206 ist mit der Kolbenstange 6 bewegungsverbunden, die über eine einer Hubhöhe bzw. einem Längsspiel 207 der Kupplungsvorrichtung 7 entsprechende Länge einen Durchmesser 208 aufweist, der im wesentlichen einem Durchmesser einer Einlaßöffnung 209 entspricht. Die Bohrung 205 ist durch Anschläge 210 begrenzt. Dieser Durchmesser 208 ist kleiner als ein Durchmesser der Zylinderkammer 13 bzw. eines Umhüllungskreises der die aus vier unter 90° zueinander ausgerichteten Wangen gebildeten Kolbenstange 6 einschließt.

Die Bohrung 205 ist in ihrem der Einlaßöffnung 209 gegenüberliegenden Endbereich durch ein Verschlußelement 211, welches einen Schwächungsbereich 212 aufweist, verschlossen. Das Verschlußelement kann dabei durch die Stirnfläche 204 des Kolbens 8 bzw. einen Bereich mit geringerer Wandstärke gebildet sein. Beidseits des Schwächungsbereiches 212 können Anschläge 213, die ebenfalls durch eine Wandstärkenverdickung in der Stirnfläche 204 des Kolbens 8 gebildet sein können, vorgesehen sein. Auf diesen Anschlägen 213 kann sich der Innenkolben 206, wenn er mit der Kolbenstange 6 in Richtung des Kolbens 8 gedrückt wird, abstützen. Der Innenkolben 206 weist weiters einen Öffnungsdorn 214 auf, der ein Öffnungsorgan 215 bildet. Dieses Öffnungsorgan ist dem Verschlußelement bzw. dessen Schwächungsbereich 212 zugeordnet.

Des weiteren ist im Kolben 8 eine Entlüftungsöffnung 216 angeordnet, über die ein Innenraum 217 der Bohrung 205 mit einer Umgebungsluft 218, die sich auch in der Zylinderkammer 13 befindet, verbunden ist.

Zur Führung kann der Kolben 8 weiters mit einem Führungsansatz 219 versehen sein, der an sich keine Abdichtung zur Innenwand 10 des Spritzenzylinders 2 bewirkt, andererseits aber verhindert, daß beim Verschieben des Kolbens 8 dieser eine Schräglage gegenüber der Längsachse 202 einnehmen kann.

Des weiteren ist der Spritzenzylinder 2 wie schematisch angedeutet, im Bereich seiner Öffnung 12 mit in die Zylinderkammer 13 vorspringenden Noppen bzw. warzenförmigen Erhöhungen 220 versehen, die auch über den Umfang des Spritzenzylinders verteilt angeordnet sein können. Diese Erhöhungen 220 sollen verhindern, daß beim Aufziehen eine in der Injektionsnadel 4 durch Striche symbolisch angedeuteten Lösung 221, beispielsweise einem Medikament oder einer Injektionsflüssigkeit oder beispielsweise auch Blut, der Kolben 8 nur nach Überwindung eines Widerstandes aus dem Spritzenzylinder 2 herausgezogen werden kann, sodaß ein versehentliches Herausziehen des Kolbens 8 möglichst vermieden wird.

Die Erhöhungen 220 dürfen jedoch nicht zu hoch ausgeführt werden, sodaß die Elastizität der Kolbendichtung bzw. der am Kolben 8 angeordneten Kolbenringe ausreicht, um den Kolben 8 von der Öffnung 12 her in den Spritzenzylinder 2 einschieben zu können.

In der in Fig.1 gezeigten Stellung wird durch ein Herausziehen der Kolbenstange 6 in Richtung eines Pfeiles 222 Lösung in die Zylinderkammer 11 angesaugt, wozu der Kolben 8 in Richtung der Öffnung 12 bewegt wird. Während dieses Ansaugens der Lösung 221 kann ein ausreichender Unterdruck in der Zylinderkammer 11 hergestellt werden, da die Stirnfläche 204 des Kolbens 8 den Spritzenzylinder 2 gegen die Öffnung 12 abdichtet.

Ist nunmehr eine ausreichende Lösungsmenge in der Zylinderkammer 11 enthalten und sollte diese beispielsweise in einen tierischen oder menschlichen Körper injiziert werden, so muß die Kolbenstange 6 über die Handhabe 203 entgegen einem Pfeil 222 in Richtung der Injektionsnadel 4 gepreßt werden. Aufgrund des Widerstandes, den die Lösung 221 einer Bewegung des Kolbens 8 in Richtung der Injektionsnadel 4 entgegenstellt, wird sich daher vorerst der Innenkolben 206 relativ zum Kolben 8 innerhalb der Bohrung 205 verschieben.

In Fig.2 ist eine im wesentlichen der Darstellung in Fig.1 entsprechende Injektionsvorrichtung 1 gezeigt, die sich von der in Fig.1 gezeigten Ausführungsform nur durch die Ausbildung des Innenkolbens 223 unterscheidet.

Bei dieser Ausführungsform wird der Innenkolben 223 gegenüber dem Kolben 8 dadurch abgedichtet, daß anstelle von umlaufenden Dichtringen in Art von Kolbenringen bzw. O-Ringen auf den Stirnseiten des Innenkolbens 223 umlaufende Dichtringe 224 angeordnet sind. Ansonsten ist die Wirkung und die Funktionsweise gleich mit der in Fig.1 dargestellten Injektionsvorrichtung. Aus dieser Darstellung in Fig.2 ist ersichtlich, daß beim Vorwärtsdrücken der Kolbenstange 6 der Innenkolben 206 bzw. 223 so weit vorwärtsbewegt wird, bis er an den beispielsweise durch Teile des Kolbens 8 gebildeten Anschlägen 213 anliegt. Bevor es zur Anlage des Kolbens 8 an diesen Anschlägen 213 kommt, durchtrennt aber die Spitze des Öffnungsdorns 214 bereits den Schwächungsbereich 212, sodaß er nach erfolgter Vorwärtsverstellung und Anlage des Innenkolbens 223 an den Anschlägen 213 die in Fig.2 gezeigte Stellung einnimmt.

In dieser Stellung kann nunmehr über die Kolbenstange 6 eine entsprechend hohe Kraft aufgebracht werden, um den gesamten Kolben 8 in Richtung der Injektionsnadel bzw. des Auslasses 5 vorwärtszuschieben, sodaß die durch kleine Striche angedeutete Lösung 221 aus der Zylinderkammer 11 ausgetragen werden kann.

Soll nunmehr entgegen den gängigen Vorschriften die Injektionsvorrichtung 1 ein zweites Mal verwendet werden, so ist es vorerst notwendig, den Innenkolben 223 aus der in Fig.2 gezeigten Stellung in die in Fig.1 gezeigte Stellung zurückzuziehen, sodaß über die Anlage des Innenkolbens 206 bzw. 223 an den Anschlägen 210 der gesamte Kolben 8 bei einer Bewegung in Richtung des Pfeiles 222 mitgenommnen werden kann um einen ausreichenden Unterdruck in der Zylinderkammer 11 zu erzeugen, sodaß eine Lösung 221 aufgenommen werden kann. Dies ist aber mit der beschriebenen Injektionsvorrichtung 1 dadurch nicht mehr möglich, da auch nach dem Zurückziehen des Öffnungsorganes 215 in die in Fig.2 in strichlierten Linien gezeichnete Stellung, das Verschlußelement, welches im Schwächungsbereich durch das Öffnungsorgan 215 durchstoßen wurde, offen bleibt und somit die Umgebungsluft 218, welche durch dünne gewellte Pfeile eingezeichnet wurde, über die Zylinderkammer 13 und die Entlüftungsöffnung 216 in die Zylinderkammer 11 eintritt und dadurch in dieser kein ausreichender Unterdruck erzeugt werden kann, um eine Lösung 221 bzw. eine Flüssigkeit aufzusaugen.

Somit ist zuverlässig verhindert, daß die erfindungsgemäße Injektionsvorrichtung 1 mehrmals verwendet wird, da durch die im Kolben angeordnete, durch das Verschlußelement 211 mit dem Schwächungsbereich 212 und dem Öffnungsdorn 214 gebildete Originalitätssicherungsvorrichtung 201 das Aufziehen einer weiteren Lösung oder Flüssigkeit zuverlässig verhindert ist.

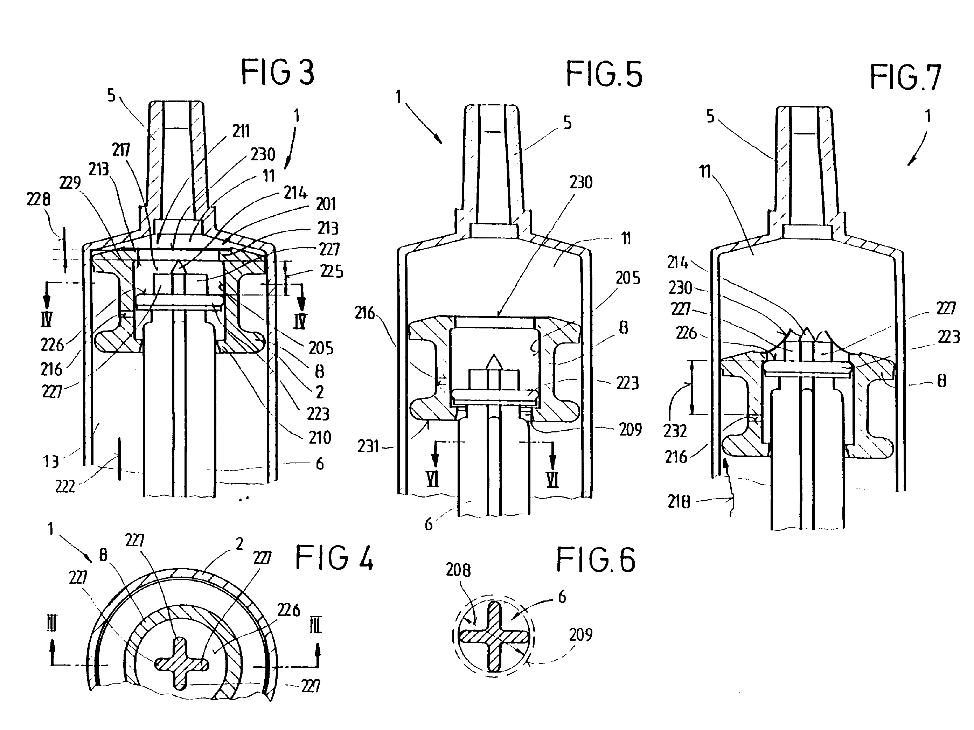

In den Fig.3 bis 7 ist eine weitere Ausführungsform einer erfindungsgemäßen Injektionsvorrichtung 1 gezeigt, die im wesentlichen gleichartig aufgebaut ist, wie die Injektionsvorrichtung 1 nach Fig.1, weshalb für gleiche Teile auch gleiche Bezugszeichen verwendet werden, sich jedoch nur hinsichtlich der Ausführung der originalitätssicherungsvorrichtung 201 von der in Fig.1 und 2 gezeigten Injektionsvorrichtung 1 unterscheidet. Auch bei dieser Ausführungsform der Injektionsvorrichtung 1 ist zusätzlich zum Kolben 8 ein mit der Kolbenstange 6 verbundener Innenkolben 223 enthalten. Die Bewegung des Innenkolbens 223 ist wieder durch Anschläge 210 bzw. 213 in beiden Richtungen begrenzt. Der Innenraum 217, der den Innenkolben 223 aufnehmenden Bohrung 205 ist wiederum über eine Entlüftungsöffnung 216 mit der durch den Kolben 8 von der Zylinderkammer 11 abgetrennten Zylinderkammer 13 im Spritzenzylinder 2 verbunden. Die Originalitätssicherungsvorrichtung 201 besteht im vorliegenden Fall wiederum aus einem Öffnungsdorn 214, der als Öffnungsorgan 215 wirkt und um eine Länge 225 über eine Stirnseite 226 des Innenkolbens 223 vorragt, wie dies ebenso in Fig.1 und 2 der Fall ist. Im vorliegenden Fall ist diese Länge 225 jedoch größer, da zwischen dem Öffnungsdorn 214 und der Stirnseite 226 Spreizorgane 227 angeordnet sind.

Die Länge 225 des Öffnungsdorns 214 und der Spreizorgane 227 ist in jedem Fall größer als eine Distanz 228 zwischen einer dem Innenkolben 223 zugeordneten Anlagefläche 229 des Anschlages 213 und der das Verschlußelement 211 für die Bohrung 205 bildenden Dichtfolie 230. Wie aus der Darstellung in Fig.4 weiters zu ersehen ist, werden die Spreizorgane 227 durch vier unter 90° zueinander angeordnete, vorspringende Lappen gebildet. Ebenso weist die Kolbenstange 6, wie besser aus Fig.6 zu ersehen ist, einen kreuz- bzw. plusförmigen Querschnitt auf. Ein Durchmesser 208 eines die Kolbenstange 6 umhüllenden Hüllkreises ist, wie aus dieser Darstellung ersichtlich, kleiner als der Durchmesser der mit strichlierten Linien in Fig.6 eingetragenen Einlaßöffnung 209 im Bereich einer Rückseite 231 des Kolbens 8.

Die Einlaßöffnung 209 kann außerdem in Richtung des Auslasses 5 konisch zusammenlaufend ausgebildet sein, sodaß das Einsetzen des Innenkolbens 223 erleichtert wird.

Die Funktion der erfindungsgemäßen Injektionsvorrichtung die in Fig.3 bis 7 gezeigt ist, ist nun folgendermaßen.

Wird der Kolben in der in Fig.3 gezeigten Stellung durch Bewegung des Innenkolbens 223 in Richtung des Pfeils 222 mitgenommen, so kann über den Auslaß 5 eine Flüssigkeit bzw. eine Lösung aufgezogen werden. Die während des Aufziehens der Flüssigkeit bzw. der Lösung vorhandene Stellung zwischen Innenkolben 223 und Kolben 8 ist am besten aus Fig.5 ersichtlich. Dabei kann ein ausreichender Unterdruck in der Zylinderkammer 11 erzeugt werden, da die Bohrung 205 durch die Dichtfolie 230 verschlossen ist.

Soll nun die aufgenommene Lösung durch den Auslaß 5 ausgepreßt werden, so muß dazu der Innenkolben 223 in die aus Fig.7 ersichtliche Relativstellung zum Kolben 8 gebracht werden. Diese Verschiebung des Innenkolbens 223 bewirkt aber, daß der Öffnungsdorn 214 die Dichtfolie 230 durchsticht, die dann durch die nachfolgenden Spreizorgane 227 soweit aufgerissen wird, daß eine ausreichend direkte Verbindung der Zylinderkammer 11 mit den zwischen der Dichtfolie 230 und der Stirnseite 226 des Innenkolbens 223 befindlichen Luftraums hergestellt wird.

Dadurch wird in einfacher Weise verhindert, daß beim Auspressen der Flüssigkeit aus der Zylinderkammer 11 nach dem Entlüften der Injektionsvorrichtung 1 noch Luftblasen mit der Flüssigkeit bzw. der Lösung mitgerissen werden können.

Ebenso, wie bereits anhand der Fig.1 und 2 beschrieben ist eine nochmalige Verwendung der Injektionsvorrichtung 1 dadurch unmöglich gemacht, daß beim Zurückziehen des Innenkolbens 223, sodaß der Kolben 8 vom Auslaß 5 wegbewegt werden kann, sich der Innenkolben 223 zuerst relativ gegenüber dem Kolben 8 in die in Fig.5 gezeigte Stellung zurückzieht, wobei jedoch das Erzeugen eines Unterdrucks in der Zylinderkammer 11 nicht mehr möglich ist, da durch die zerstörte Dichtfolie 230 Luft über die Entlüftungsöffnung 216 in die Zylinderkammer 11 eindringen kann.

Auch bei dieser Injektionsvorrichtung 1 ist es ebenso, wie bei den Injektionsvorrichtungen in Fig.1 und 2 möglich, durch die Wahl eines Längsabstandes 232 ein Volumen vorzudefinieren, um welches der Innenkolben 223 relativ zum Kolben 8 verstellt werden kann, ohne daß Umgebungsluft 218 in die Zylinderkammer 11 eintreten kann. Dieser Längsabstand 232 bzw. der Hub des Innenkolbens 223 kann zum sogenannten Aspirieren verwendet werden. Dieser Vorgang ist notwendig, um festzustellen, ob beim Einstechen der Injektionsvorrichtung 1 in den tierischen bzw. menschlichen Körper eine Vene getroffen wurde, sodaß bei Lösungen bzw. Medikamenten, die direkt in die Blutbahn injiziert werden müssen, dies sofort überprüft werden kann. Andererseits ist es bei solchen Medikamenten, die nicht in die Blutbahn injiziert werden dürfen, dadurch möglich, festzustellen, ob tatsächlich keine Vene getroffen wurde oder nicht.

Bei diesen beschriebenen Vorrichtungen bedarf es jedoch eines gefühlvollen Umganges mit der Injektionsvorrichtung 1, da ansonsten bei einem Überschreiten der Entlüftungsöffnung 216 durch den Innenkolben 223 wiederum Umgebungsluft 218 in die Zylinderkammer 11 eintreten kann. In diesem Fall muß der Injektionsvorgang nochmals unterbrochen werden, um die Luft wiederum aus der Zylinderkammer 13 zu entfernen.

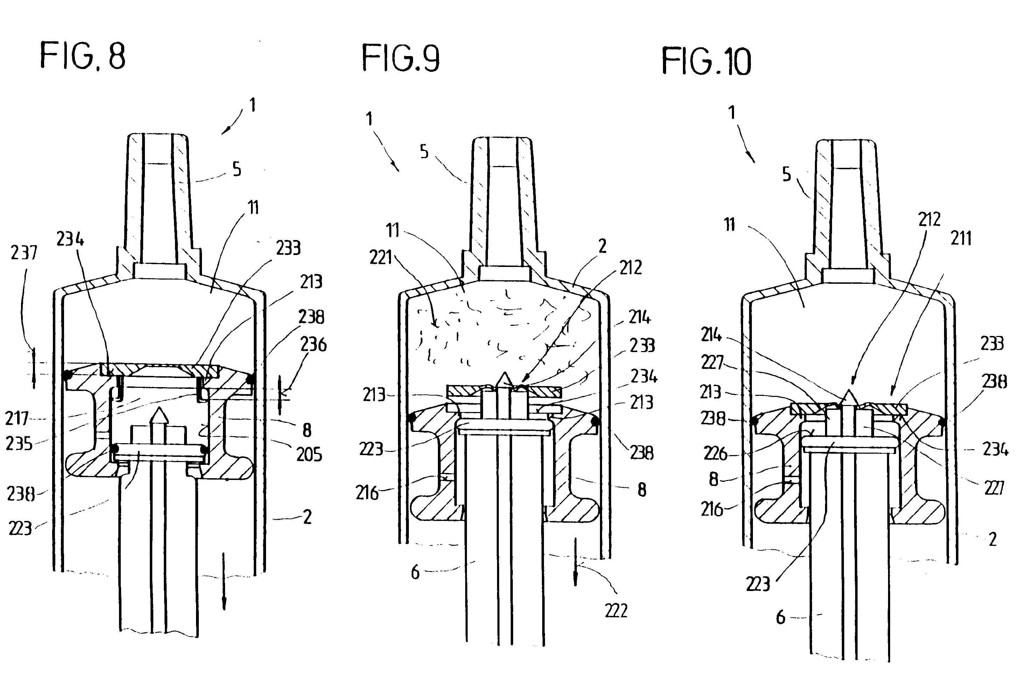

In den Fig.8 bis 10 ist eine andere Ausführungsform einer Injektionsvorrichtung 1 bei unterschiedlichen Stellungen des Kolbens 8 bzw. des Innenkolbens 223 gezeigt, wobei für gleiche Teile wiederum gleiche Bezugszeichen verwendet werden. Bei dieser Ausführungsform ist der Innenraum 217 der Bohrung 205 im Kolben 8 gegenüber der Zylinderkammer 11 durch eine Dichtscheibe 233, die das Verschlußelement 211 bildet, abgedichtet. Diese Dichtscheibe 233 ist in eine Ausnehmung 234 eingesetzt und zumindest leicht eingepreßt. Überdies kann sie mit Halteorganen 235 versehen sein, welche die Anschläge 213 um eine Distanz 236, die größer ist als eine Tiefe 237 der die Dichtscheibe 233 aufnehmenden Ausnehmung 234, überragen. Diese Halteorgane 235 können, wie später noch erläutert werden wird, verhindern, daß auch bei einem Lösen der Dichtscheibe 233 diese in die Zylinderkammer 11 hineinfallen und gegebenenfalls den Auslaß 5 blockieren.

Wie weiters aus den Darstellungen in den Fig.8 bis 10 zu ersehen ist, kann die Abdichtung zwischen dem Kolben 8 bzw. Innenkolben 223 sowie zwischen dem Kolben 8 und dem Spritzenzylinder 2 durch Kolbenringe 238 bzw. durch O-Ringe oder sonstige Dichtungselemente erfolgen. Durch die Anordnung der Dichtscheibe 233 soll nun bei der erfindungsgemäßen Injektionsvorrichtung 1 eine Möglichkeit geschaffen werden, das Aspirieren so zu ermöglichen, daß nach dem Ansetzen der Injektionsvorrichtung 1 bzw. dem Einstechen der Injektionsnadel 4 in den tierischen bzw. menschlichen Körper mit hoher Wahrscheinlichkeit keine Luft mehr in die Zylinderkammer 11 eintreten kann. Während nun, wie bereits anhand der vorstehenden Ausführungsbeispiele beschrieben, durch Zurückbewegen des Kolbens 8 in Richtung des Pfeiles 222 eine Flüssigkeit bzw. eine Lösung angesaugt werden kann, ist zum Auspressen diese Lösung 221 aus der Zylinderkammer 11 der Innenkolben 223 in die in Fig.9 gezeigte Stellung zu verstellen. Da eine Kraftübertragung von der Kolbenstange 6 auf den Kolben 8 erst dann erfolgen kann, wenn der Innenkolben 223 an den Anschlägen 213 anliegt, wird, bevor eine Bewegung des Kolbens 8 in Richtung des Auslasses 5 erfolgt, die Dichtscheibe 233 von dem Öffnungsdorn 214 wie in Fig.9 und 10 gezeigt, durchstochen. Dadurch, daß das Material auch in einem Schwächungsbereich 212 der Dichtscheibe 233 ausreichend steif ausgeführt ist und die Dichtscheibe 233 in die Ausnehmung 234 ausreichend fest eingesetzt ist, durchdringt der Öffnungsdorn 214 die Dichtscheibe 233 und spießt diese zwischen dem Öffnungsdorn 214 und den Spreizorganen 227 auf. Damit wird die Dichtscheibe, wie in Fig.9 gezeigt, aus der Ausnehmung 234 hinausgedrückt und abgehoben. Dieser Zustand wird erreicht beim Entlüften der Spritze, bei welchem eine gewisse Menge an Lösung 221 aus der Zylinderkammer 11 ausgepreßt werden muß. Gleichzeitig kann dann in dieser Lage die Entlüftung der Zylinderkammer 11 erfolgen. Dies wird üblicherweise derart vorgenommen, daß der Arzt oder die Schwester, die die Injektion verabreichen durch Klopfen an der Spritze die Luftblasen in den Bereich des Auslasses 5 verbringen, worauf dann so viel Lösung 221 ausgespritzt wird, bis die Luftblasen durch die Nadel ausgetreten sind.

Ist nun die Injektionsvorrichtung 1 entlüftet, so wird die Injektionsnadel 4 in den Körper eingestochen und daraufhin mit der Kolbenstange 6 der Innenkolben 223 gegenüber dem Kolben 8 soweit zurückgezogen, bis die Dichtscheibe 233 die in Fig.10 gezeigte Stellung einnimmt. Ist nun die Festigkeit der Rastverbindung zwischen dem Öffnungsdorn 214 und der Dichtscheibe 233 ausreichend groß, so kann damit auch der Kolben 8 über die Kolbenstange 6 ein geringes Stück zurückgezogen werden, bevor sich beispielsweie der Öffnungsdorn 214 von der Dichtscheibe 233 löst. Es ist aber andererseits ebenso möglich, die Distanz zwischen dem Öffnungsdorn 214 und der diesem zugewandten Stirnseite 226 des Innenkolbens 223 so groß auszulegen, daß durch die Bewegung des Innenkolbens 223 eine ausreichende Menge an Flüssigkeit angesaugt werden kann, sodaß in dem transparenten Teil der Injektionsvorrichtung 1, nämlich dem Spritzenzylinder 2 erkennbar ist, ob eine Vene getroffen wurde oder nicht. Dies ist dadurch ersichtlich, ob beim Zurückziehen der Kolbenstange 6 Blut in die Zylinderkammer 11 eingesaugt wird oder nicht. Daraufhin kann durch ein Bewegen der Kolbenstange 6 entgegen des Pfeiles 222, in dem dann der Innenkolben 223 die aus Fig.9 ersichtliche Relativstellung zum Kolben 8 einnimmt, die benötigte Flüssigkeitsmenge bzw. die Lösung 221 aus der Zylinderkammer 11 in den Körper injiziert werden. Zu berücksichtigen ist dabei noch, daß die Entlüftungsöffnung 216 derart anzuordnen ist, daß bei dem in der Dichtscheibe 233 gehaltenen Öffnungsdorn 214 und in die Ausnehmung 234 eingesetzter Dichtscheibe 233 der Innenkolben 223 noch zwischen dieser Entlüftungsöffnung 216 und den Anschlägen 213 liegt, da ansonsten wiederum die Gefahr bestehen würde, daß durch die Entlüftungsöffnung 216 Luft in die Zylinderkammer 11 eindringen könnte. Somit ist durch diese Lösung eine Möglichkeit geschaffen, daß bei einer erfindungsgemäßen Injektionsvorrichtung unter Einhaltung sämtlicher medizinischer Vorschriften ein Aspirieren möglicht ist und trotzdem bei dem Versuch, nach dem Ausspritzen der Lösung 221 eine weitere Lösung anzusaugen, aufgrund der dadurch benötigten Kraft der Innenkolben 223 aus der Dichtscheibe 233 heraustritt und seine in Fig.8 gezeigte Stellung einnimmt. Durch die zerstörte Dichtscheibe 233 wird jedoch dann über die Entlüftungsöffnung 216 beim weiteren Zurückziehen der Kolbenstange 6 kein Vakuum mehr in der Zylinderkammer 11 aufgebaut, und es kann keine Lösung 221 mehr angesaugt werden.

Selbstverständlich ist es möglich, die Gestaltung der Dichtscheibe 233 bzw. des Verschlußelementes 211 oder des Öffnungsdorns 214 beliebig abzuändern. So können unter anderem auch mehrere Öffnungsdorne 214 beispielsweise auf den einzelnen Flügeln bzw. Spreizorganen 227 angeordnet sein, bzw. können schlankere Spitzen oder schneidenartige Organe oder dgl. ebenso Verwendung finden. Wesentlich ist, daß das Durchtrennen der Schwächungsbereiche 212 ohne allzu großen Kraftaufwand erfolgen kann, sodaß bei jenem Kraftaufwand, der notwendig ist, um eine Lösung 221 aus der Zylinderkammer 11 auszupressen, das Verschlußelement 211 zuverlässig zerstört wird und damit eine Wiederverwendung der Injektionsvorrichtung 1 von vorne herein ausgeschalten wird.

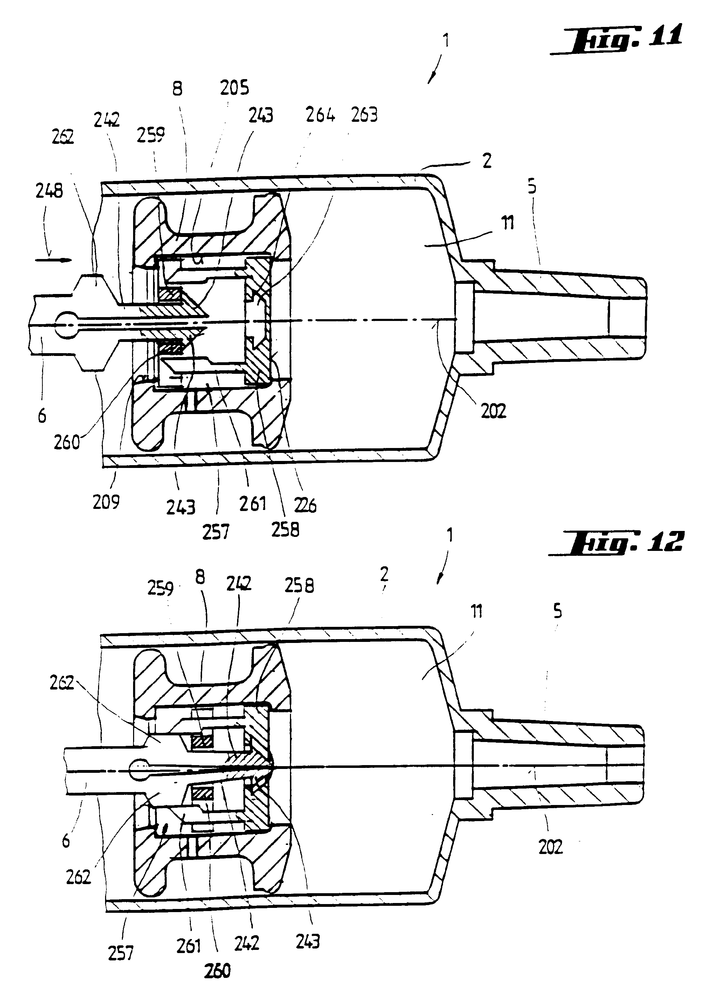

In den Fig.11 und 12 ist eine Lösung gezeigt, bei der ein im Kolben 8 angeordneter Innenkolben 257 aus zwei Teilen 258 und 259 besteht. Der Teil 258 ist scheibenartig ausgebildet und liegt dichtend an der Bohrung 205 im Kolben 8 an. Der Teil 259 besteht aus einer kreuzförmigen Platte, die eine Öffnung 260 aufweist, die von Rastarmen 242 durchsetzt ist, die an der Kolbenstange 6 angeformt bzw. mit dieser bewegungsverbunden sind. Die Rastarme 242 weisen radial nach außen gerichtete Arretiernasen 243 auf, die den Teil 259 von der den Teil 258 zugewandten Seite her untergreifen. Desweiteren ist der Teil 258 mit Steuerarmen 261 versehen, die in etwa eine der Länge der Bohrung 205 entsprechende Länge aufweisen. Die balkenförmigen Arme des als Stützplatte dienenden Teils 259 des Innenkolbens 257 sind jeweils zwischen zwei solcher Steuerarme 261 angeordnet. Auf den Rastarmen 242 sind Steuerleisten 262 in einem Abstand, der geringer ist als eine Länge der Bohrung 205 angeordnet. Die Steuerleisten 262 weisen einen Hüllkreisdurchmesser auf, der in etwa dem Durchmesser der Einlaßöffnung 209 zur Bohrung 205 entspricht.

Der Teil 258 ist im Bereich einer Stirnseite 226 flächendichtend ausgeführt und weist auf der den Teil 259 zugewandten Seite eine Ausnehmung 263 mit Hinterschneidungen 264 auf, die zur Aufnahme der Arretiernasen 243 der Rastarme 242 ausgebildet sind. Die zur Verwendung vorbereitete Injektionsvorrichtung 1 wird nun derart geliefert, daß der Teil 259 lose auf den Teil 258 des Innenkolbens 257 aufliegt und die Rastarme mit ihren Arretiernasen 243 nur den Teil 259 hintergreifen. Soll nun mit der Injektionsvorrichtung eine entsprechende Menge von Lösung 221 aufgenommen werden, so wird die Kolbenstange 6 entgegen der Richtung des Pfeils 248 zurückgezogen, wodurch der Teil 259 den Kolben 8 mitnimmt, und dementsprechend in der Zylinderkammer 11 ein Vakuum entsteht, über welches die Lösung angesaugt wird. Ist eine entsprechende Menge an Lösung in der Injektionsvorrichtung 1 enthalten, so kann durch ein Vorwärtsschieben der Kolbenstange 6 in Richtung des Pfeils 248 über den Teil 258 ein Druck auf den Kolben 8 ausgeübt werden, der ein Auspressen von Lösungen durch den Auslaß 5 bewirkt. Durch den dabei ausgeübten Druck treten die Rastarme 242 in die Ausnehmung 263 ein und deren Arretiernasen 243 schnappen in die Hinterschneidung 264 ein. Wird nun die Kolbenstange 6 beispielsweise beim Aspirieren wieder in eine dem Pfeil 248 entgegengesetzte Richtung zurückgezogen, so bewegt sich der Teil 258 mit den Steuerarmen 261 ebenfalls in Richtung der Einlaßöffnung 209. Die Steuerarme 261 werden dabei in der Einlaßöffnung 209 zusammengedrückt. Laufen nun die Steuerleisten 262 bei einer weiteren Bewegung der Kolbenstange 6 entgegen des Pfeils 248 auf die Steuerarme 261 auf, so werden dadurch die Rastarme 242 in Richtung der Längsachse 202 radial nach innen gedrückt, sodaß sie aus den Hinterschneidungen 264 heraustreten und die Arretiernasen 243 einen kleineren Hüllkreisdurchmesser aufweisen, als der Durchmesser der Öffnung 260 im Teil 259 des Innenkolbens 257. Wird nun weiterhin eine entgegen der Richtung des Pfeils 248 gerichtete Kraft auf die Kolbenstange 6 ausgeübt, so wird die Kolbenstange 6 lose herausgezogen, ohne daß sich der Kolben 8 weiter mitbewegt. Somit kann bis zum Auftreten eines etwas höheren Widerstandes, nämlich dann, wenn die Steuerleisten 262 auf die Steuerarme 261 auflaufen, ein Zurückziehen zum Aspirieren erfolgen, wobei der zum Aspirieren notwendige Freiraum durch das Zurückziehen des Innenkolbens 257 in der Bohrung 205 geschaffen wird. Aber selbst für den Fall, daß durch ein zu heftiges Zurückziehen der Kolbenstange 6 bereits während des Aspiriervorganges die Kolbenstange 6 vom Kolben 8 herausgezogen wird, ist es möglich, das Wiedereinschieben der Kolbenstange 6 in den Kolben 8 in Richtung des Pfeils 248 vorzunehmen, wobei jedoch ein Wiedereinkuppeln verhindert ist. Aber selbst für den Fall, daß die Kolbenstange 6 zufällig so eingeführt wird, daß sie erneut mit den Teilen 258 und 259 in eine Rastverbindung tritt, wird die Kolbenstange 6 bei einem erneuten Zurückbewegen gegen die Richtung des Pfeils 248 durch das Zusammenwirken der Steuerleisten 262 mit den Steuerarmen 261 zuverläßig entkuppelt.

Demgemäß ist auch mit dieser Ausführungsform eine Injektionsvorrichtung geschaffen, mit der ein Aspirieren möglich ist, jedoch eine gezielte zweimalige Verwendung der Injektionsvorrichtung ausgeschalten wird.

Selbstverständlich können die zu den einzelnen Ausführungsbeispielen beschriebenen Teile auch fallweise bei den anderen Ausführungsbeispielen eingesetzt werden, und es ist überdies möglich, die Kolben 8 bzw. die Innenkolben 206, 223 und 257 in jeder beliebigen aus dem Stand der Technik bekannten Bauweise auszubilden. Vor allem ist zu berücksichtigen, daß die einzelnen Teile, die Innenkolben 206, 223 und 257 und Kolben 8 während der Schiebung eine entsprechende Betätigungskraft erfordern und andererseits die Verstellung der Rastarme 242 bzw. das Zerstechen des Verschlußelementes 211 einen nur geringen Kraftaufwand erfordern, sodaß nicht durch die zur Betätigung der vorgenannten Elemente benötigten Kräfte ausreichen, um den Kolben 8 relativ zum Spritzenzylinder 2 zu verschieben.

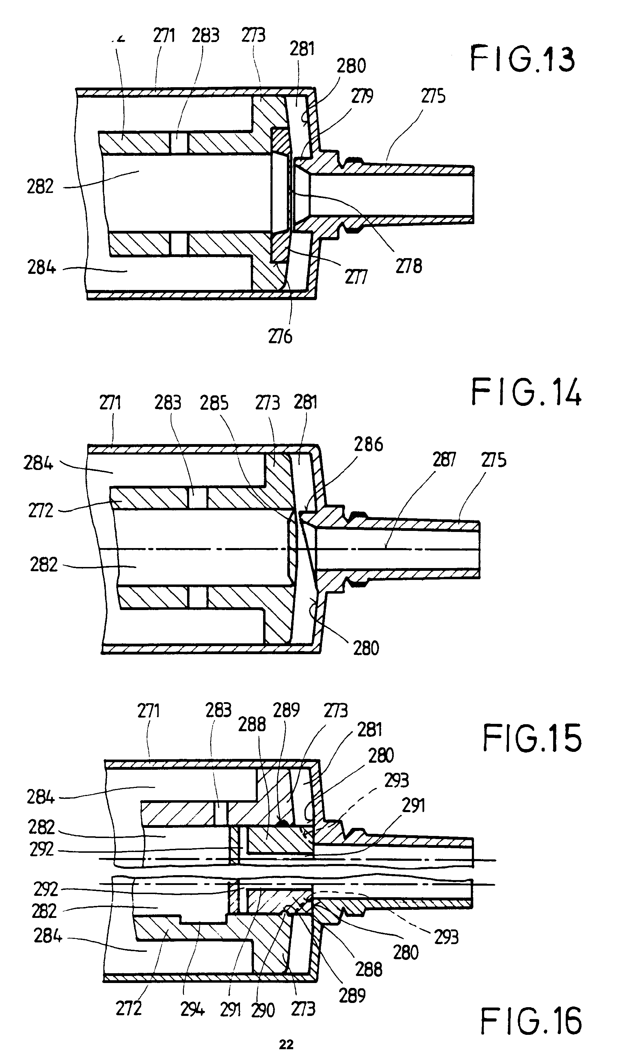

In Fig.13 ist mit 271 der Spritzenzylinder einer Injektionsvorrichtung bzw. Einwegspritze bezeichnet, in welchem ein Kolben 272 geführt ist, wobei die Kolbenstirnfläche 273 dichtend an der Innenfläche des Spritzenzylinders 271 angreift. Zur Führung des Kolbens 272 im Spritzenzylinder 271 können dabei nicht näher dargestellte Führungsrippen am Außenumfang des Kolbens 272 vorgesehen sein. Der Spritzenzylinder 271 weist einen Spritzenkonus 275 auf, auf welchen vor einer Verwendung der Spritze eine Kanüle aufsteckbar ist.

Der Kolben 272 weist an seiner Kolbenstirnfläche 273 einen abgesetzten Bereich 276 auf, in welchen ein Dichtungselement 277 als gesonderter Bauteil eingepreßt ist. Dieses Dichtungselement 277 wird dabei von einem Plättchen oder einer Scheibe gebildet, welches eine Folie 278 aus Kunststoff oder elastomerem Material trägt, welches nicht näher dargestellte Sollbruchstellen aufweist. Vor einem Aufziehen von zu injizierenden pharmazeutischen Mitteln wird der Kolben 272 bis in die in Fig.13 dargestelle Position bewegt, d.h. bis in die Anlage des Dichtungselementes 277 mit der Folie 278 an einen am kolbenseitigen Ende des Spritzenkonus 275 vorgesehenen Anschlag 279, worauf das flüssige, zu injizierende Mittel aufgezogen werden kann. Für eine vollständige Verabreichung der Injektion muß nunmehr darauf geachtet werden, daß die Kolbenstirnfläche 273 des Kolbens 272 bis in Anlage an die den Spritzenkonus 275 tragende Stirnwand 280 gelangt, wodurch die Folie 278 vom Anschlag 279 zerstört wird. Durch die Zerstörung der Folie 278, welche entlang nicht näher dargestellter Sollbruchstellen aufreißt, wird in der Folge eine Verbindung zwischen dem Raum 281 vor der Kolbenstirnfläche und dem in axialer Richtung verlaufenden Hohlraum 282 im Inneren des Kolbens 272 geöffnet, sodaß ein neuerliches Dosieren durch Aufziehen eines pharmazeutischen Mittels durch Füllung des Raumes 281 vor dem Kolben unmöglich wird. Eine weitergehende Sicherung gegen eine neuerliche Verwendung kann dabei dadurch vorgenommen werden, daß der Kolben radiale Durchbrechungen 283 aufweist, wodurch eine Verbindung des Raumes 281 über den axialen Hohlraum 282 hinter der Kolbenstirnfläche 273 mit dem Kolbenstangenraum 284 geschaffen wird, welcher zum vom Spritzenkonus 275 abgewandten Ende des Spritzenzylinders 271 im allgemeinen offen ausgebildet ist, sodaß ein neuerliches Aufziehen nach der Zerstörung der Folie 278 verhindert wird.

Bei der in Fig.14 dargestellten Ausführungsform ist das Dichtungselement zum axialen Hohlraum 282 des Kolbens 272 als flächenbündige Scheibe 285 an der Kolbenstirnfläche 273 angeordnet, welche an ihrem, an die Kolbenstirnfläche 273 anschließenden Umfangsbereich verjüngt ausgebildet ist. Als Anschlag wird bei dieser Ausführungsform eine an der Stirnwand 280 des Spritzenzylinders 271 vorgesehene Schneide 286 verwendet, welche mit der Stirnwand 280 starr verbunden ist und eine schräg zur Achse 287 des Kolbens bzw. des Spritzenzylinders verlaufende Schneidkante aufweist. Durch diese schräg verlaufende Schneide wird sichergestellt, daß bei Vollendung des Spritzenhubes zum Austragen des pharmazeutischen Mittels die Scheibe 285 an einem Punkt entlang ihres Umfanges sicher aufgetrennt wird und somit wiederum eine Verbindung zwischen dem Raum 281 vor der Kolbenstirnfläche 273 mit dem axialen Hohlraum 282 bzw. über radiale Durchbrechungen 283 mit dem Kolbenstangenraum 284 vorliegt, sodaß eine Weiterverwendung der Spritze verhindert wird.

Bei den Darstellungen gemäß Fig.15 und 16 sind die Bezugszeichen für gleiche Bauteile beibehalten worden. Als Dichtungselement wird bei diesen Ausführungsformen ein im axialen Hohlraum 282 des Kolbens 272 geführter Dichtungskolben 288 eingesetzt, welcher in seiner in den Fig.15 und 16 dargestellten Dichtungslage im Spritzenkolben über die Kolbenstirnfläche 273 des Kolbens 272 vorragt und wird in dieser Stellung, beispielsweise durch ein Zusammenwirken einer Ringnut 289 mit einem Ringwulst 290 gehalten, welche ein jeweils einander entsprechendes Profil aufweisen und am Umfang des Dichtungskolbens 288 bzw. im axialen Hohlraum 282 angeordnet sind. Vor einem Aufziehen des pharmazeutischen Mittels wird der Kolben 272 in die in den Fig.15 und 16 dargestellten Position gebracht, in welcher der Dichtungskolben 288 den Raum 281 vor der Kolbenstirnfläche vom Hohlraum 282 im Kolben abdichtet. Beim Ausbringen des pharmazeutischen Mittels wird der Dichtungskolben 288 durch die als Anschlag wirkende Stirnwand 280 in das Innere des axialen Hohlraumes 282 des Kolbens 272 verschoben, wodurch eine Verbindung zwischen dem vor der Kolbenstirnfläche liegenden Raum 281 und dem axialen Hohlraum 282 bzw. dem Kolbenstangenraum 284 über einen axialen Kanal 291 und wenigstens einen radialen Kanal 292 im Dichtungskolben erfolgt. Bei Beendigung des Spritzenhubes liegt die Stirnfläche des Dichtungskolbens 288 etwa auf Höhe der Kolbenstirnfläche 273 des Kolbens 272, sodaß bei der in Fig.15 dargestellten Ausführungsform eine Verbindung zwischen dem Raum 281 und die Kanäle 291 und 292 in fluchtender Anordnung mit der oder den Durchbrechungen 283 mit dem Kolbenstangenraum 284 geschaffen wird. Um eine derartige offene Verbindung zwischen dem Raum vor der Kolbenstirnfläche und dem Kolbenstangenraum 284 sicherzustellen, können die radialen Kanäle 292 in eine Ringnut am Umfang des Dichtungskolbens 288 münden, sodaß die offene Verbindung in jeder Drehlage des Kolbens 272 sichergestellt ist. Um ein vollkommenes Austragen des pharmazeutischen Mittels sicherzustellen, weist dabei der Dichtungskolben 288 in seinem über die Kolbenstirnfläche 273 vorragenden Bereich an seinem Umfang oder seiner Stirnfläche Ausnehmungen oder Nuten auf, wie sie durch die strichlierten Linien 293 angedeutet sind.

Bei der in Fig.16 dargestellten Ausführungsform ist abweichend von der Ausbildung gemäß Fig.15 wenigstens eine Ausnehmung 294 an der Innenfläche des hohlen Kolbens 272 vorgesehen, sodaß auch bei dieser Ausbildung nach einem Verschieben des Dichtungskolbens in eine im wesentlichen bündige Lage mit der Kolbenstirnfläche 273 über die Kanäle 291 und 292 und eventuell über eine bei der Mündung der Kanäle 292 vorgesehene Ringnut am Umfang des Dichtungskolbens eine offene Verbindung zwischen dem Raum 281 vor der Kolbenstirnfläche 273 und dem axialen Hohlraum 282 im Inneren des Kolbens 272 sichergestellt wird.

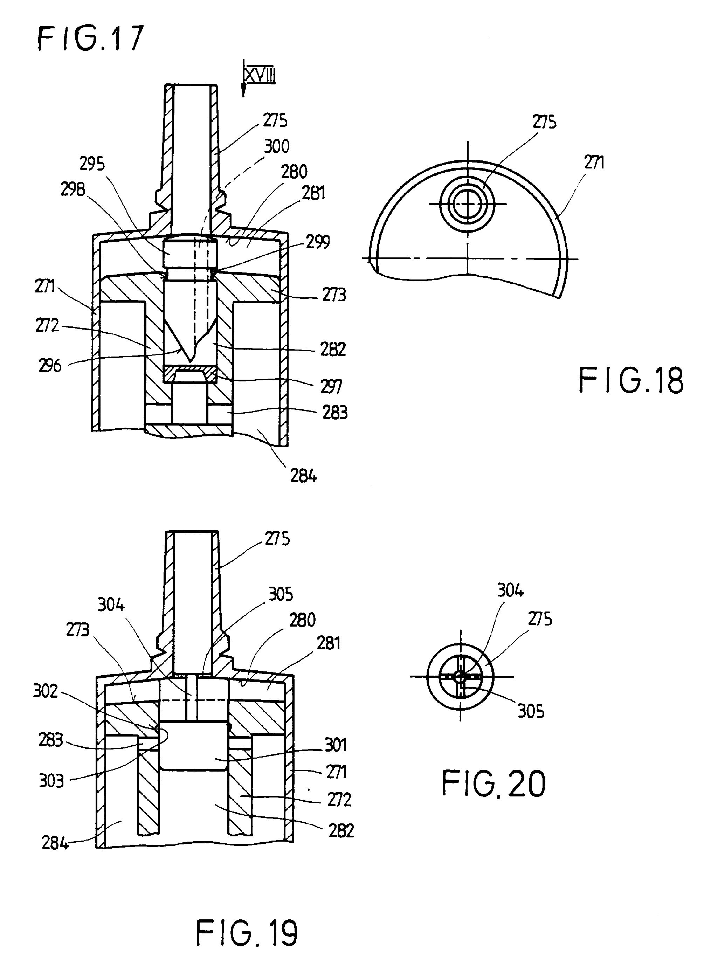

Bei der in Fig.17 dargestellten Ausführungsform der Einwegspritze ist im axialen Hohlraum 282 des Kolbens 272 ein Anschlag 295 verschieblich geführt, welcher an seinem dem Spritzenkonus 275 abgewandten Ende eine Schneide 296 trägt, welche beim vollkommenen Einschieben des Kolbens 272 in den Spritzenzylinder 271 durch eine Verschiebung des mit der Stirnwand 280 zusammenwirkenden Anschlages 295 eine Zerstörung des im Abstand von der Kolbenstirnfläche 273 angeordneten Dichtelementes 297 ergibt. Der Anschlag 295 wird dabei über einen Ringwulst 298, welcher in eine Umfangsnut 299 des Anschlages 295 eingreift in einer über die Kolbenstirnfläche 273 vorragenden Position in seiner Lage gehalten. Für ein sicheres Ausbringen des gesamten im Raum 281 vor der Kolbenstirnfläche 273 enthaltenen Volumens weist der Anschlag 295 an seiner dem Spritzenkonus 275 zugewandten Stirnfläche wiederum Ausnehmungen bzw. eine Nut auf, welche der Übersichtlichkeit halber nicht dargestellt ist. Nach einem Verschieben des Anschlages 295 mit seiner Schneide 296 in Richtung zum Dichtelement 297 und der Zerstörung des Dichtelementes 297 ist wiederum über einen strichliert angedeuteten, im wesentlichen in axialer Richtung verlaufenden Kanal 300 im Inneren des Anschlages 295 eine offene Verbindung zwischen dem Raum 281 und dem axialen Hohlraum 282 sowie über die Durchbrechungen 283 mit dem Kolbenstangenraum 284 sichergestellt, sodaß eine weitere Verwendung verhindert wird.

Bei der in Fig.18 dargestellten Ansicht auf die Einwegspritze gemäß der Fig.17 ist deutlich die exzentrische Anordnung des Spritzenkonus 275 am Spritzenzylinder 271 ersichtlich für welche die Ausführungsform des Anschlages und des Dichtungselementes gemäß der Fig.17 besonders günstig ist.

Bei der in Fig.19 dargestellten Ausführungsform ist im axialen Hohlraum 282 des Kolbens 272 wiederum ein Dichtungskolben 301 verschieblich geführt, welcher in seiner Ausgangslage beispielsweise über einen in eine Ringnut 302 eingreifenden Ringwulst 303 am Umfang des Dichtungskolbens gehalten ist. Der Anschlag wird bei dieser Ausführungsform von einem Dorn 304 gebildet, welcher über radiale Stege 305 im Bereich der Stirnwand 280 des Spritzenzylinders 271 abgestützt ist. Durch die Abstützung des Dornes 304 über die radialen Stege 305 ist somit ein ausreichender Durchtrittsquerschnitt vom Raum 281 in den Spritzenkonus 275 und die daran anschließende Kanüle sichergestellt. Bei Verabreichung des zu injizierenden Mittels bewirkt der Dorn 304 eine Verschiebung des Dichtungskolbens 301 im axialen Hohlraum 282 des Kolbens 272, wobei nach einer Anlage der Kolbenstirnfläche 273 an der Stirnwand 280 der Dichtungskolben 301 soweit verschoben wurde, daß eine offene Verbindung zwischen dem Raum 281 vor der Kolbenstirnfläche und dem Kolbenstangenraum 284 über die radialen Durchbrechungen 283 im Mantel des Kolbens sichergestellt wird. Für die Herstellung einer derartigen Verbindung zwischen dem Raum 281 vor der Kolbenstirnfläche 273 und dem axialen Hohlraum 282 bzw. dem Kolbenstangenraum 284 kann der Kolben auch mit im Inneren angeordneten axialen und radialen Kanälen versehen sein, wie dies in den Fig.15 und 16 dargestellt ist.

In Fig.20 ist schematisch mit 275 die Außenkontur des Spritzenkonus bezeichnet und es ist die Abstützung des Dornes 304 über an der Stirnwand 280 vorgesehene radiale Stege 305 näher veranschaulicht.

Ein Dorn 304, wie er in den Fig.19 und 20 dargestellt ist, kann jedoch auch bei einer Ausbildung eines Dichtelementes in Form einer Folie Verwendung finden, welche in Abstand von der Kolbenstirnfläche analog zur Stirnfläche des Dichtungskolbens 301 angeordnet sein kann, wobei die Folie durch ein Durchstechen unbrauchbar gemacht werden kann.

Bei sämtlichen Ausführungsformen ergibt sich somit nach einem vollkommenen Einschieben des Kolbens 272, d.h. bei einer Anlagestellung der Kolbenstirnfläche 273 an die Stirnwand 280 entweder eine Zerstörung des Dichtelementes zum axialen Hohlraum 282 bzw. zum Kolbenstangenraum 284 oder es wird der Dichtungskolben 288 bzw. 301 in eine derartige und nicht rückstellbare Position verschoben, in welcher jeweils eine offene Verbindung zwischen dem Raum 281 vor der Kolbenstirnfläche und dem Raum hinter der Kolbenstirnfläche bzw. dem axialen Hohlraum 282 und bzw. oder dem Kolbenstangenraum 284 jeweils sichergestellt wird, wodurch eine neuerliche Verwendung der Einwegspritze verhindert wird.

# B e z u g s z e i c h e n a u f s t e l l u n g

1 Injektionsvorrichtung
2 Spritzenzylinder
3 Kupplungsanordnung
4 Injektionsnadel
5 Auslaß

6 Kolbenstange
7 Kupplungsvorrichtung
8 Kolben
9
10 Innenwand

11 Zylinderkammer
12 Öffnung
13 Zylinderkammer
201 Originalitätssicherungsvorrichtung
202 Längsachse

203 Handhabe
204 Stirnfläche
205 Bohrung
206 Innenkolben
207 Längsspiel

208 Durchmesser
209 Einlaßöffnung
210 Anschlag
211 Verschlußelement
212 Schwächungsbereich

213 Anschlag
214 Öffnungsdorn
215 Öffnungsorgan
216 Entlüftungsöffnung
217 Innenraum

218 Umgebungsluft
219 Führungsansatz
220 Erhöhung
221 Lösung
222 Pfeil

223 Innenkolben
224 Dichtring
225 Länge
226 Stirnseite
227 Spreizorgan

228 Distanz
229 Anlagefläche
230 Dichtfolie
231 Rückseite
232 Längsabstand

233 Dichtscheibe
234 Ausnehmung
235 Halteorgan
236 Distanz
237 Tiefe

238 Kolbenring
242 Rastarm
243 Arretiernase
248 Pfeil
257 Innenkolben

258 Teil
259 Teil
260 Öffnung
261 Steuerarm
262 Steuerleiste

263 Ausnehmung
264 Hinterschneidung
271 Spritzenzylinder
272 Kolben
273 Kolbenstirnfläche

274
275 Spritzenkonus
276 Bereich
277 Dichtungselement
278 Folie

279 Anschlag
280 Stirnwand
281 Raum
282 Hohlraum
283 Durchbrechung

284 Kolbenstangenraum
285 Scheibe
286 Schneide
287 Achse
288 Dichtungskolben

289 Ringnut
290 Ringwulst

291 Kanal
292 Kanal
293 Linie
294 Ausnehmung
295 Anschlag

296 Schneide
297 Dichtelement
298 Ringwulst
299 Umfangsnut
300 Kanal

301 Dichtungskolben
302 Ringnut
303 Ringwulst
304 Dorn
305 Steg

**Patentansprüche**

1. Injektionsvorrichtung (1) mit einem Spritzenzylinder (2), einem in dem Spritzenzylinder (2) geführten Kolben (8) und einer mit diesem insbesondere über eine Kupplungsvorrichtung verbundenen Kolbenstange (6), die bevorzugt eine Länge aufweist, die größer ist als die Länge des Spritzenzylinders (2), mit einer auf der von der Kolbenstange (6) abgewendeten Seite des Spritzenzylinders (2) vorgesehenen Kupplungsanordnung (3), über die eine Injektionsnadel (4) mit dem Spritzenzylinder (2) kuppelbar ist und mit einer Originalitätssicherungsvorrichtung (201), die ein zwischen Kolben (8) und der Kolbenstange (6) angeordnetes, durch einen Öffnungdorn (214) gebildetes Öffnungsorgan (215) und der Kolben (8) eine parallel zur Längsachse des Spritzenzylinders (2) verlaufende Bohrung (205) aufweist, dadurch gekennzeichnet, daß in der Bohrung (205) ein mit der Kolbenstange (6) bewegungsverbundener Innenkolben (206,223) gelagert ist und die Bohrung (205) sowohl in Richtung der Kolbenstange (6) als auch in Richtung der Zylinderkammer (11) durch Kupplungselemente der Kupplungsvorrichtung (7) bildende Anschläge begrenzt ist, die in den Querschnitt der Bewegungsbahn des Innenkolbens (206,223) hineinragen, sowie auf der der Zylinderkammer (11) zugewandten Stirnseite durch ein Verschlußelement (211) verschlossen und in einem in Richtung der Kolbenstange (6) distanzierten Bereich über eine Entlüftungsöffnung (216) mit der Umgebungsluft (218) verbunden ist und daß der Öffnungsdorn (214) über eine dem Verschlußelement (211) zugewandte Stirnseite (226) des Innenkolbens (206,223) vorragt.

2. Injektionsvorrichtung nach Anspruch 1, dadurch gekennzeichnet, daß bei einer an den der Kolbenstange (6) zugewandten Anschläge (213) anliegenden Stellung des Innenkolbens (206,223) die Entlüftungsöffnung (216) zwischen dem Innenkolben (206,223) und dem Verschlußelement (211) angeordnet ist.

3. Injektionsvorrichtung nach Anspruch 1 oder 2, dadurch gekennzeichnet, daß das Verschlußelement (211) durch eine Dichtfolie (230) oder einen Schwächungsbereich (212) in einer Stirnfläche (204) des Kolbens (8) gebildet ist.

4. Injektionsvorrichtung nach einem oder mehreren der Ansprüche 1 bis 3, dadurch gekennzeichnet, daß das Verschlußelement (211) durch eine insbesondere kunststoffbeschichtete Aluminiumfolie gebildet ist.

5. Injektionsvorrichtung nach einem oder mehreren der Ansprüche 1 bis 4, dadurch gekennzeichnet, daß die Dichtscheibe (233) in eine Ausnehmung (234) in die Stirnfläche (204) des Kolbens (8) eingesetzt ist.

6. Injektionsvorrichtung nach einem oder mehreren der Ansprüche 1 bis 5, dadurch gekennzeichnet, daß der Schwächungsbereich (212) zentrisch insbesondere im Bereich der Längsachse (202) des Spritzenzylinders (2) im Verschlußelement (211) angeordnet ist.

7. Injektionsvorrichtung nach einem oder mehreren der Ansprüche 1 bis 6, dadurch gekennzeichnet, daß der Öffnungsdorn (214) konzentrisch zur Längsachse (202) des Spritzenzylinders (2) bzw. des Innenkolbens (206,223) oder der Kolbenstange (6) angeordnet ist.

8. Injektionsvorrichtung nach einem oder mehreren der Ansprüche 1 bis 3, dadurch gekennzeichnet, daß dem Öffnungsdorn (214) in radialer Richtung benachbart Spreizorgane (227) zugeordnet sind, die ebenfalls über die Stirnseite (226) des Innenkolbens (206,223) vorstehen.

9. Injektionsvorrichtung nach einem oder mehreren der Ansprüche 1 bis 8, dadurch gekennzeichnet, daß auf der der Kolbenstange (6) zugewandten Seite des Kolbens (8) und bzw. oder des Innenkolbens (206,223) ein Führungsansatz (219) vorgesehen ist.

10. Injektionsvorrichtung nach einem oder mehreren der Ansprüche 1 bis 9, dadurch gekennzeichnet, daß der Kolben (8) mit Kolbenringen (238) versehen ist.

11. Injektionsvorrichtung nach einem oder mehreren der Ansprüche 1 bis 10, dadurch gekennzeichnet, daß zwischen dem Innenkolben (206,223) und der Bohrung (205) Kolbenringe (238) angeordnet sind.

12. Injektionsvorrichtung nach einem oder mehreren der Ansprüche 1 bis 11, dadurch gekennzeichnet, daß das Verschlußelement (211) Halteorgane (235) umfaßt, die die dem Verschlußelement (211) nächstliegenden Anschläge (213) hintergreifen, wobei bei in die Ausnehmung (234) eingesetzten Verschlußelement (211) eine Distanz (236) zwischen den Halteorganen (235) und den Anschlägen (213) größer ist als eine Tiefe (237) der Ausnehmung (234).

13. Injektionsvorrichtung nach einem oder mehreren der Ansprüche 1 bis 12, dadurch gekennzeichnet, daß die Vorsprünge in einer Distanz von der Rückseite (231) des Kolbens (8) angeordnet ist und an den Vorsprung eine sich in Richtung der Rückseite konisch erweiternde Stützfläche für die Rastarme (242) anschließt, wobei ein Teil der der Rückseite (231) des Kolbens (8) näherer Stützflächenteil der Stützfläche auf einem unabhängig von diesem verstellbar gelagerten Spreizring angeordnet ist, der eine Vertiefung insbesondere eine Nut oder eine Hinterschneidung für die Arretiernasen (243) der Rastarme (242) aufweist.

14. Injektionsvorrichtung nach einem oder mehreren der Ansprüche 1 bis 13, dadurch gekennzeichnet, daß der Innenkolben (257) zweiteilig ist und die Kolbenstange (6) Rastarme (242) umfaßt, die eine Stützplatte des Innenkolbens (257) durchsetzen und dessen Arretiernasen (243) im Kolbenteil eine Vertiefung zugeordnet ist, und daß die Rastarme (242) Steuerleisten aufweisen die radial nach außen um eine Distanz vorspringen, daß ein diese umfassender Hüllkreis in etwa einen Durchmesser aufweist, der einem Durchmesser der Durchgangsöffnung des Kolbens entspricht und daß am Kolbenteil sich in Richtung der Kolbenstange (6) erstreckende Steuerarme (261) angeordnet sind, die zumindest eine Länge aufweisen, die größer ist als ein Abstand zwischen den Arretiernasen (243) und den Steuerleisten (262).

15. Injektionsvorrichtung nach einem oder mehreren der Ansprüche 1 bis 14, dadurch gekennzeichnet, daß in radialer Richtung eine Dicke der Steuerleisten (262) größer ist als eine Rasttiefe der Arretiernasen (243).

16. Injektionsvorrichtung nach einem oder mehreren der Ansprüche 1 bis 15, dadurch gekennzeichnet, daß sich die Durchgangsöffnung in Richtung des Innenkolbens (257) kegelförmig erweitert.

17. Injektionsvorrichtung nach einem oder mehreren der Ansprüche 1 bis 16, dadurch gekennzeichnet, daß eine Länge (225) des über die Stirnseite (226) vorspringenden Öffnungsorganes (215) größer ist als eine Distanz (228) zwischen der Stirnseite (226) des Innenkolbens (206,223) zugeordneten Anlageflächen (229) der Anschläge (213) und dem Verschlußelement (211) insbesondere des Schwächungsbereiches (212) parallel zur Längsachse (202) des Spritzenzylinders (2).

## Claims

1. Injection device (1) with a syringe cylinder (2), a piston (8) guided in the syringe cylinder (2) and a piston rod (6) connected to the piston particularly by a coupling device, the length of the piston rod preferably exceeding that of the syringe cylinder (2), and further provided with a coupling arrangement (3) at the side of the syringe cylinder facing away from the piston rod (6) for coupling an injection needle (4) to the syringe cylinder (2), and with an originality protection device (201) comprising an opening member (215) in the form of an opening spike (214) arranged between the piston (8) and the piston rod (6) and the piston (8) containing a bore (205) that extends parallel to the logitudinal axis of the syringe cylinder (2), characterized in that an inner piston (206, 223) is positioned in the bore (205), motionally coupled to the piston rod (6) and in that the bore (205) is both, delimited in the direction of the piston rod (6) as well as in the direction of the cylinder chamber (11) by coupling elements of the coupling device (7) forming limiting stops that project inwardly into the cross-section of the path of the movement of the inner piston (206, 223), that are closed on the end face facing the cylinder chamber (11) by a sealing element (211) and connected to the ambient atmosphere (218) by means of a venting aperture (216) in a region remote from the direction of the piston rod (6) and that the opening spike (214) projects from the end face (226) of the inner piston (206, 223) towards the sealing element (211).

2. Injection device according to claim 1, characterized in that when the inner piston (206, 223) is in a position in contact with the limiting stops (213) facing towards the piston rod (6), the venting aperture (216) is arranged between the inner piston (206, 223) and the sealing element (211).

3. Injection device according to claim 1 or 2, characterized in that the sealing element (211) is formed by a sealing foil (230) or a weakened section (212) in an end face (204) of the piston (8).

4. Injection device according to one or several of claims 1 to 3, characterized in that the sealing element (211) is formed by an aluminium foil, particularly one coated with plastics.

5. Injection device according to one or several of claims 1 to 4, characterized in that the sealing disk (233) is inserted in a recess (234) in the end face (204) of the piston (8).

6. Injection device according to one or several of claims 1 to 5, characterized in that the weakened section (212) is arranged centrally in the sealing element (211), particularly in the region of the longitudinal axis (202) of the syringe cylinder (2).

7. Injection device according to one or several of claims 1 to 6, characterized in that the opening spike (214) is arranged concentrically with the longitudinal axis (202) of the syringe cylinder (2) respectively the inner piston (206, 223) or the piston rod (6).

8. Injection device according to one or several of claims 1 to 3, characterized in that radially adjacent spreading elements (227) are associated with the opening spike (214) that also project beyond the end face (226) of the inner piston (206, 223).

9. Injection device according to one or several of claims 1 to 8, characterized in that a guiding projection (219) is provided at the side of the piston (8) and/or the inner piston (206, 223) facing towards the piston rod (6).

10. Injection device according to one or several of claims 1 to 9, characterized in that the piston (8) is provided with piston rings (238).

11. Injection device according to one or several of claims 1 to 10, characterized in that piston rings (238) are arranged between the inner piston (206, 223) and the bore (205).

12. Injection device according to one or several of claims 1 to 11, characterized in that the sealing element (211) comprises retaining members (235) that engage behind the limiting stops (213) closest to the sealing element (211), and when the sealing element (211) is inserted in the recess (234) the distance (236) between the retaining members (235) and the limiting stops (213) is greater than the depth (237) of the recess (213).

13. Injection device according to one or several of claims 1 to 12, characterized in that the projections are arranged at a spacing from the rear end (231) of the piston (8) and that widening out conically in the direction of the rear end the projection is adjoined by a supporting surface for the locking arms (242), whereby that part of a supporting surface section of the supporting surface that is nearer to the rear end (231) of the piston (8) is arranged on a spreader ring, positioned displaceably, independently thereof, which contains a recess, particularly a groove or an undercut portion for the arresting lugs (243) of the locking arms (243).

14. Injection device according to one or several of claims 1 to 13, characterized in that the inner piston (206, 223) consists of two parts and the piston rod (6) comprises locking arms (242) that extend through a supporting plate of the inner piston (257) and its arresting lugs (243), that in turn are associated with a cavity in a part of the piston, and that the locking arms (242) have control members which project some distance radially outwardly, that an enveloping circle covering these members approximately has a diameter corresponding to that of the passage through the piston and that control arms (261) extending in the direction of the piston rod (6) are arranged on the piston part, and that they are at least longer than the space between the arresting lugs (243) and the control members (262).

15. Injection device according to one or several of claims 1 to 14, characterized in that in a radial direction the thickness of the control members (262) is greater than the engaging depth of the arresting lugs (243).

16. Injection device according to one or several of claims 1 to 15, characterized in that the passage towards the inner piston is extended in a cone shaped way.

17. Injection device according to one or several of claims 1 to 16, characterized in that a length (225) of the opening member (215) projecting beyond the end face (226) is greater than a distance (228) between the contact surfaces (229) on the limiting stops (213) associated with the end face (226) of the inner piston (206, 223) and the sealing element (211), particularly of the weakened section (212) parallel with the longitudinal axis (202) of the syringe cylinder (2).

## Revendications

1. Dispositif d'injection (1) avec un cylindre de seringue (2), un piston (8) guidé dans le cylindre de seringue (2) et en particulier, relié à celui-ci par l'intermédiaire d'un dispositif d'accouplement une tige de piston (6) dont la longueur est de préférence plus grande que la longueur du cylindre de seringue (2), avec une disposition d'accouplement (3) prévue sur le côté du cylindre de seringue (2) tourné à l'opposé de la tige de piston (6), par laquelle une aiguille d'injection (4) est accouplable avec le cylindre de seringue (2), et avec un dispositif de sécurité d'origine (201) qui présente entre le piston (8) et la tige de piston (6) un organe d'ouverture (215) constitué par un goujon de percement (214) et le piston (8) présentant une forure (205) parallèlement à l'axe longitudinal du cylindre de seringue (2), caractérisé en ce que dans la forure (205) est logé un piston intérieur (206, 223) relié de façon mobil à la tige de piston (6) et en ce que la forure (205) est délimitée non seulement en direction de la tige de piston (6) mais encore en direction de la chambre du cylindre (11) par des éléments d'accouplement du dispositif d'accouplage (7) formant des butées, qui s'étendent jusqu'à l'intérieur de la section transversale de la voie de mouvement du piston intérieur (206, 223), ainsi qu'elle est fermée au moyen d'un élément de fermeture (211) sur la face orientée vers la chambre du cylindre (11), et qu'elle est reliée au moyen d'un évent (216) à l'air ambiant (218) dans une zone espacée de la direction de la tige de piston (6) et que le goujon de percement (214) s'étend en saillie au-delà de la face (226) du piston intérieur (206, 223) tournée vers l'élément de fermeture (211).

2. Dispositif selon la revendication 1, caractérisé en ce que dans une position adjacente du piston intérieur (206, 223) sur les butées tournées vers la tige de piston (6), l'évent (216) est disposé entre le piston intérieur (206, 223) et le dispositif de fermeture (211).

3. Dispositif d'injection selon la revendication 1 ou 2, caractérisé en ce que l'élément de fermeture (211) est constitué par une feuille pour étanchement (230) ou par une partie affaiblie (212) sur une face (204) du piston (8).

4. Dispositif d'injection selon une ou plusieurs des revendications 1 à. 3, caractérisé en ce que l'élément de fermeture (211) est constitué par une feuille d'aluminium présentant en particulier une couche de matière synthétique.

5. Dispositif d'injection selon une ou plusieurs des revendications 1 à 4, caractérisé en ce que le disque d'étanchéité (233) est inséré dans un creux (234) sur la face (204) du piston (8).

6. Dispositif d'injection selon une ou plusieurs des revendications 1 à 5, caractérisé en ce que la partie affaiblie (212) est disposée centralement, en particulier au voisinage de l'axe longitudinal (202) du cylindre de seringue (2) dans l'élément de fermeture (211).

7. Dispositif d'injection selon une ou plusieurs des revendications 1 à 6, caractérisé en ce que le goujon de percement (214) est disposé concentriquement sur l'axe longitudinal (202) du cylindre de seringue (2), du piston intérieur (206, 223) ou de la tige de piston (6).

8. Dispositif d'injection selon une ou plusieurs des revendications 1 à 3, caractérisé en ce qu'au voisinage s'étendant radialement du goujon de percement (214) sont associés des organes à expansion (227), qui s'étendent en saillie également de la face (226) du piston intérieur (206, 223).

9. Dispositif d'injection selon une ou plusieurs des revendications 1 à 8, caractérisé en ce qu'un embout de guidage (219) est prévu sur le côté du piston (8) et/ou du piston intérieur (206, 223) tourné vers la tige de piston (6).

10. Dispositif d'injection selon une ou plusieurs des revendications 1 à 9, caractérisé en ce que le piston (8) est pourvu de segments de piston (238).

11. Dispositif d'injection selon une ou plusieurs des revendications 1 à 10, caractérisé en ce que les segments de piston (238) sont disposés entre le piston intérieur (206, 223) et la forure (205).

12. Dispositif d'injection selon une ou plusieurs des revendications 1 à 11, caractérisé en ce que l'élément de fermeture (211) comprend des organes de retenue (235), s'engrénant en arrière des butées voisines à l'élément de fermeture (211), ledit élément de fermeture (211) étant inséré dans le creux (234) de manière que la distance (236) entre les organes de retenue (235) et les butées (213) soit plus grande que la profondeur (237) du creux (234).

13. Dispositif d'injection selon une ou plusieurs des revendications 1 à 12, caractérisé en ce que les saillies sont disposées à une distance du dos (231) du piston (8) et qu'il s'ajoute à la saillie en direction du dos une surface d'appui s'étendant coniquement pour les bras d'encliquetage (242), à quoi une partie d'une section de la surface d'appui de la surface d'appui qui est plus près au dos (231) du piston (8) est disposée sur un anneau d'écartement, logé de manière déplaçable, indépendamment du piston, qui présente une dépression, en particulier une rainure ou un contre-dépouille pour les ergots d'arrêt (243) des bras d'encliquetage (242).

14. Dispositif d'injection selon une ou plusieurs des revendications 1 à 13, caractérisé en ce que le piston intérieur (257) est composé de deux pièces et la tige de piston (6) comprend des bras d'encliquetage (242), qui transpercent une plaque d'appui du piston intérieur (257) et dont les ergots d'arrêt (243) dans la partie du piston sont associés à une dépression, et que les bras d'encliquetage (242) présentent des barres de commande qui s'étendent radialement vers l'extérieur sur une distance afin que le diamètre de la gaine enveloppant soit à peu près égal au diamètre de l'ouverture de passage du piston et que sur la partie du piston sont disposés des bras d'encliquetage (261) s'étendant en direction de la tige de piston (6), qui présentent au moins une longueur plus grande qu'un écartement entre les ergots d'arrêt (243) et les barres de commande (262).

15. Dispositif d'injection selon une ou plusieurs des revendications 1 à 14, caractérisé en ce que l'épaisseur des barres de commande (262) en direction radiale est plus grande que la profondeur d'encliquetage des ergots d'arrêt (243).

16. Dispositif d'injection selon une ou plusieurs des revendications 1 à 15, caractérisé en ce que l'ouverture de passage en direction du piston intérieur (257) s'étend en forme de cône.

17. Dispositif d'injection selon une ou plusieurs des revendications 1 à 16, caractérisé en ce qu'une longueur (225) de l'organe d'ouverture (2115) s'étendant en saillie sur la face (226) est plus grande qu'une distance (228) entre les surfaces de contact (229) des butées (213) associées à la face (226) du piston intérieur (206, 223) et l'élément de fermeture (211), en particulier de la partie affaiblie (212) s'étendant parallèlement à l'axe longitudinal (202) du cylindre de seringue (2).

17

FIG.1

FIG. 2

FIG.7

FIG.5

FIG.6

FIG.3

FIG.4

# FIG. 8

# FIG. 9

# FIG. 10

EP 0 438 453 B1

_Fig. 11_

_Fig. 12_

FIG.13

FIG.14

FIG.15

FIG.16

22

# FIG.17

# FIG.18

# FIG.19

# FIG.20